# EUROPEAN PATENT SPECIFICATION

(11) **EP 1 733 736 B1**
(45) Date of publication and mention of the grant of the patent: **25.02.2015**
(21) Application number: 06014768.3
(22) Date of filing: 07.02.2002
(51) Int. Cl.: A61K 39/395, A61K 47/48, A61P 35/00, C07K 16/30

(54) **Method of producing recombinant antibodies against tumours**
Verfahren zur Herstellung von rekombinanten Antikörpern gegen Tumore
Procédé de production des anticorps recombinants contre des tumeurs

(30) Priority: 07.02.2001 US 266853 P; 05.10.2001 US 327008 P
(43) Date of publication of application: 20.12.2006
(62) Divisional of application: 02704692.9
(73) Proprietor: Wilex AG, 81675 München (DE)
(72) Inventor: Oosterwijk, Egbert, Dr., 6641 Beuningen (NL); Warnaar, Sven, Prof., 2334 Leiden (NL); Ullrich, Stefan, Dr., 82319 Starnberg (DE)
(74) Representative: Weiss, Wolfgang

(56) References cited:
- M. STEFFENS ET AL.: "Phase I radioimmunotherapy of metastatic renal cell carcinoma with 131I-labeled chimeric monoclonal antibody G250.", CLINICAL CANCER RESEARCH, vol. 5, 1 October 1999 (1999-10-01), pages 3268S-3274S, XP002615900,
- H. UEMURA ET AL.: "MN/CA IX/G250 as a potential target for immunotherapy of renal cell carcinomas.", BRITISH JOURNAL OF CANCER, vol. 81, no. 4, October 1999 (1999-10), pages 741-746, XP002300254,
- M. STEFFENS ET AL.: "Targeting of renal cell carcinoma with iodine-131-labeled chimeric monoclonal antibody G250.", JOURNAL OF CLINICAL ONCOLOGY, vol. 15, no. 4, April 1997 (1997-04), pages 1529-1537, XP009061594,
- J. VAN DIJK ET AL.: "Therapeutic effects of monoclonal antibody G250, interferons and tumor necrosis factor, in mice with renal-cell carcinoma xenografts.", INTERNATIONAL JOURNAL OF CANCER, vol. 56, no. 2, 15 January 1994 (1994-01-15), pages 262-268, XP008023392,
- PENICHET M L ET AL: "Antibody-cytokine fusion proteins for the therapy of cancer.", JOURNAL OF IMMUNOLOGICAL METHODS 1 FEB 2001, vol. 248, no. 1-2, 1 February 2001 (2001-02-01), pages 91-101, ISSN: 0022-1759

## Description

The invention relates to the hybridoma cell G250 or any progeny cell thereof capable of producing G250 antibody.

The fusion of mouse myeloma cells with the spleen cells from immunized mice, first described by Kohler and Milstein, Nature, 256, 495-997 (1975), makes it possible to obtain continuous cell lines which produce homogeneous antibodies, referred to as monoclonal antibodies (mAb).

Many examples exist where hybrid cells or hybridomas are described. These hybridomas are used to produce antibodies useful for various scientific investigations (Current Topics in Microbiology and Immunology, volume 81 - "Lymphocyte Hybridomas", F. Melchers et al., Springer-Verlag (1978) and references therein; C.J. Barnstable et al., Cell, (1978), 14, 9-20; P. Parham, W.F. Bodmer, Nature (1978), 276, 397-399; Handbook of Experimental Immunology, 3rd edition, vol. 2, D.M. Wier, editor, Blackwell, 1978, Chapter 25, Chem. Eng. News, 15-17 (1979); Kennett, R.H., McKearn, J.T., and Bechtol, K.B. (1980) Monoclonal Antibodies. Hybridomas: A New Dimension in Biological Analysis (Plenum, New York)). These reports describe the principal techniques for the production of monoclonal antibodies by hybridomas.

The monoclonal antibody G250, subclass IgG1, recognizes an antigen preferentially expressed on membranes of renal cell carcinoma cells (RCC) and not expressed in normal proximal tubular epithelium. The antibody G250 was obtained by immunizing a mouse with cell homogenates from primary RCC lesions obtained from different patients (Oosterwijk et al., Int. J. Cancer 38 (1986), 489-494).

The monoclonal antibody G250 as well as chimeric derivatives thereof have been used in clinical studies (Steffens et al., J. Clin. Oncol. 15 (1997), 1529-1537; Steffens et al., Clinical Cancer Research 5 (1999), 32681-32741). The nucleic acid sequence coding for the antigen-binding site of G250 is subject matter of co-pending US application No. 60/266 853, which is herein incorporated by reference.

The production of a hybridoma cell line expressing G250 antibody was generally described in the international patent application WO88/08854 and Oosterwijk et al. (supra). As stated above, a cell homogenate from primary RCC lesions obtained from different patients and thus an unspecific material was used as an immunogen. Furthermore, the hybridoma cell line had not been deposited with a recognized depository institution according to the Budapest Treaty. Thus, an exact reproduction of the G250 hybridoma cell line from the publically available prior art documents does not seem to be possible.

Meanwhile it has been found that the G250 antibody binds to the so-called MN antigen which is described e.g. in WO93/18152. The G250 binding site on the MN antigen is, however, not known at present. Moreover, recent results show that the G250 binding site is a conformational epitope which further increases the burden to reproduce the G250 hybridoma cell line, since no specified epitope sequence on the MN antigen which binds to G250 can be provided.

Thus, the present invention relates to a hybridoma cell capable of producing a G250 monoclonal antibody. This hybridoma cell was deposited under the Budapest Treaty for the Deposit of Microorganisms on September 11, 2001 at Deutsche Sammlung von Mikroorganismen und Zellkulturen GmbH (DSMZ), Mascheroder Weg 1b, 38124 Braunschweig, Germany under the Accession Number DSM ACC 2526. The deposit is the first publically available disclosure of a G250 antibody producing hybridoma cell line.

A further aspect of the invention is a progeny cell derived from said hybridoma DSM ACC 2526 which produces a G250 antibody.

In a preferred embodiment, the present invention relates to a progeny cell of the deposited hybridoma cell producing a G250 antibody wherein the progeny cell is obtained by recombinant DNA methods, e.g. by transferring genetic material encoding the G250 antibody or at least the antigen-binding site thereof into a receptor cell. The genetic material may be directly or indirectly obtained from the deposited hybridoma cell G250. "Directly obtained" means that the G250 genetic material is derived from the deposited hybridoma cell. "Indirectly obtained" means that the G250 genetic material is derived from an already existing G250 progeny cell or from other sources including chemical synthesis.

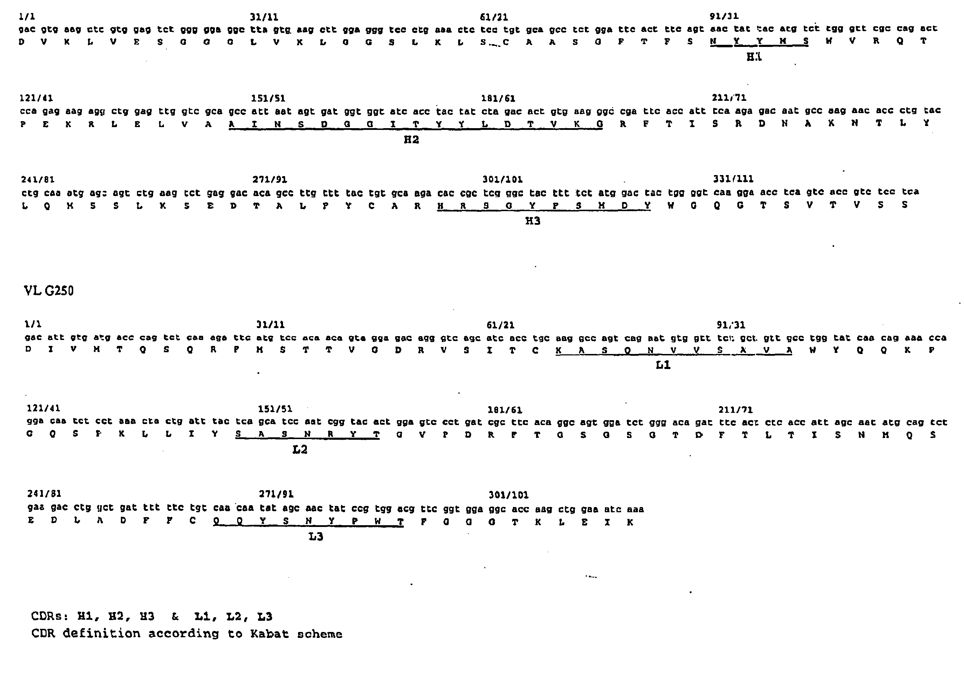

Preferably the G250 genetic material comprises nucleotide sequences encoding at least the G250 antigen-binding site, particularly nucleotide sequences encoding the complementary determining regions CDR3, CDR2 and/or CDR1 of the heavy chain antigen-binding site as shown in Figure 1 (designated H1-H3) and/or the complementary determining regions CDR3, CDR2 and/or CDR1 of the light chain antigen-binding site (designated L1-L3) as shown in Figure 1.

According to the invention the term "G250 antibody" covers any antibody including multispecific antibodies (e.g. bispecific antibodies) and antibody fragments as long as they exhibit the desired activity, i.e. at least one G250 antigen-binding site. The antibody may be an IgM, IgG (e.g. IgG₁, IgG₂, IgG₃ or IgG₄), IgD, IgA or IgE, particularly IgG antibody, a recombinant antibody or an antibody fragment obtained by proteolytic methods or by recombinant DNA methods.

The term "monoclonal antibody" as used herein refers to an antibody obtained from a population of substantially homogeneous antibodies, i.e., the individual antibodies comprising the population are identical, except for possibly naturally occurring mutations that may be present in minor amounts.

The term "antibody" as used herein refers to any polypeptide containing at least one G250 antigen-binding site, i.e. at least the CDR3 region of the G250 heavy chain and/or the CDR3 region of the G250 light chain or a variant G250 CDR3 region having an identity of at least 80%, preferably at least 90% to the original G250 CDR3 region on the amino acid level, provided that the variant CDR3 region has equivalent antigen-binding characteristics, particularly affinity and specificity compared to the original CDR3 region.

Preferably, the term "antibody" herein includes chimeric antibodies, humanized and fully humanized antibodies, single chain antibodies, e.g. sFv antibody fragments, diabody fragments, proteolytic or recombinant antibody fragments such as Fv-, Fab-, Fab'- or F(ab')₂-fragments or other antigen-binding subsequences of antibodies. The antibody may also be a fusion or a conjugate with other entities.

The antibodies herein specifically include chimeric antibodies in which a portion of the heavy and/or light chain including the antigen-binding site is identical with or homologous to corresponding sequences derived from the original hybridoma cell line G250, while the remainder of the chains is identical with or homologous to corresponding sequences derived from other species or belonging to another antibody class or subclass as well as fragments of such antibodies as long as they exhibit the desired biological activity. More preferably, the chimeric antibody comprises variable regions, e.g. the complement-determining regions (CDRs) and and the framework regions from the heavy chain and the light chain of the original G250 monoclonal antibody and constant human sequences, particularly constant human kappa light chain and gamma heavy chain sequences. The manufacture of chimeric antibodies is described e.g. by Morrison et al. (Proc. Natl. Acad. Sci. USA 81 (1984), 6851-6855), which is herein incorporated by reference.

Further, antibody herein specifically includes humanized antibodies or fully human antibodies. Humanized antibodies are immunoglobulins, immunoglobulin chains or fragments thereof which contain minimal sequence derived from non-human immunoglobulin. More particularly, humanized antibodies are human immunogiobuiins in which residues from a CDR of a given human antibody are replaced by residues from the G250 CDR, particularly the CDR1, 2 and/or 3 region of the heavy and/or light chain. Furthermore, humanized antibodies may comprise residues which are found neither in the recipient human antibody, nor in the imported G250 CDR sequences. These modifications are made to further refine and optimize antibody performance. In general, the humanized or fully human antibody will comprise substantially all of at least 1, and typically 2, variable domains, in which all or substantially all of the CDR regions correspond to those of the original G250 immunoglobulin and all or substantially all of the framework regions and constant regions are those of a human immunoglobulin sequence. The manufacture of humanized antibodies is described, e.g. in Jones et al. (Nature 321 (1986), 522-525), Riechmann et al. (Nature 332 (1988), 323-329 and Presta (Curr. Op. Struct. Biol. 2 (1992), 332-339), which are herein incorporated by reference.

Further, antibodies specifically include single-chain antibodies such as single-chain Fv antibody fragments comprising the VH and VL domains of an antibody, wherein these domains are present in a single polypeptide chain. Generally, the Fv polypeptide further comprises a polypeptide linker between the VH and VL domains which enables the sFv to form the desired structure for antigen binding. The manufacture of sFv antibodies is described e.g. by Plückthun in: The Pharmacology of Monoclonal Antibodies, Vol. 113, Rosenburg and Moore, Eds., Springer Verlag, NY, pp. 269-315 (1994), Barbas III (Methods: Companion Methods Enzymol. 2 (1991), 119) and Hoogenboom et al. (Immunol. Rev. 130 (1992), 41-68), which are herein incorporated by reference.

Further, antibodies specifically include diabodies, i.e small antibody fragments with two antigen-binding sites, which fragments comprise a heavy chain variable domain connected to a light chain variable domain in the same polypeptide chain. By using a linker that is too short to allow pairing between the two domains on the same chain, the domains are forced to pair with the complementary domains of another chain and create two antigen binding sites. The manufacture of diabodies is described e.g. by Hollinger et al., (Proc. Natl. Acad. Sci. USA 90 (1993), 6444-6448), which is herein incorporated by reference.

Further, antibodies specifically include multispecific antibodies e.g. heterobispecific antibodies comprising at least one G250 antigen-binding site and the antigen-binding site from a different antibody, e.g. an anti-CD3-antibody.

The antibody produced by the G250 hybridoma cell or a progeny cell thereof may be fused or coupled to a marker or effector component. For example, the G250 antibody may be recombinantly modified so that it is linked to cytokines such as interleukin-2 (IL-2), tumor necrosis factor (TNF) and/or granulocyte macrophage colony stimulating factor (GM-CSF).

Furthermore, the G250 antibody may be conjugated, e.g. by covalent coupling or fused to a suitable marker group, e.g. a fluorescent group, a radioactive marker group etc. or a cytotoxic agent including radioactive isotopes such as I, Y, Pr, Tm, In, such as ¹²³I, ¹²⁵I, ¹³⁵I, ^{99m}Tm or ¹¹¹In, chemotherapeutic agents and toxins such as enzymatically active toxins of bacterial, fungal, plant or animal origin, or fragments thereof.

The receptor cell into which G250 specific genetic material from the original G250 hybridoma cell or any progeny thereof or of synthetic origin is transferred may be any suitable host cell capable of expressing antibodies. For example, the host cell maybe a prokaryotic cell, preferably an E. coli cell or a eukaryotic cell. Preferred eukaryotic cells are e.g. insect, yeast and mammalian cells. Most preferred host cells are mammalian cells, e.g. human or mouse myeloma cells or CHO-cells.

Further, the present invention comprises a method of producing G250 antibody or derivatives thereof comprising:
cultivating a G250 hybridoma cell or a progeny cell thereof under suitable conditions, wherein a G250 antibody is produced and obtaining the antibody and/or derivative thereof from the cell and/or from the cell culture medium.

The antibody obtained according to this method is particularly useful for producing pharmaceutical formulations which comprise the antibody as an active agent, besides pharmaceutically acceptable carriers, diluents and/or adjuvants.

In an especially preferred embodiment, the active agent is a chimeric G250 antibody which may be present as such or as a conjugate with a radioactive group, e.g. with ¹²³I, ¹²⁵I, ¹³¹I, ^{99m}Tm or ¹¹¹In.

Further, the present invention relates to the use of the deposited hybridoma cell and progeny cells thereof for manufacturing G250 antibodies as described above, e.g. monoclonal antibodies, chimeric antibodies, humanized antibodies, fully humanized antibodies, bispecific antibodies and antibody fragments such as (Fab')₂-, Fab'-, Fab- and Fv-fragments.

The chimeric G250 antibody was successfully used in clinical studies for the treatment of renal cell carcinoma patients after surgery. Even though the study has not yet been completed, the treated patients already show a significant increase in median survival (more than 15 months) compared to untreated patients (three months) or patients treated with standard therapy (10-12 months).

Surprisingly, it was found that in some cases tumor regression occurred more than six months after start of therapy. Thus, chimeric G250 antibody and other G250 antibodies are capable of eliciting a delayed immune response in cancer therapy, preferably in the treatment of renal cell carcinoma and more preferably for the treatment of metastases after tumor surgery.

### Example 1

### Deposit of the G250 hybridoma cell line

The G250 hybridoma cell line was produced as described in Example 1 of WO88/08854. Therein a general immunization protocol is given. Further informations, e.g. a molecular characterization of the G250 antibody and the G250 hybridoma cell are lacking.

The G250 hybridoma cell line was deposited according to the requirements of the Budapest Treaty at DSMZ under the accession No. DSM ACC 2526 on September 11, 2001.

### Example 2

### Mapping of the epitope recognized by monoclonal antibody G250

From collective experimental evidence it is assumed that the G250 protein epitope recognized by monoclonal antibody G250 (MAbG250) is conformational, most likely of a non-linear nature. This assumption is based on the following observations:
- MabG250 does not react with antigen G250 in Western blot analysis Purification of G250 antigen by MAbG250 affinity chromatography is highly inefficient, with efficiencies decreasing with increased time needed to perform the purification, suggesting rapid degradation/ unfolding of the G250 epitope, in spite of optimal conditions to prevent proteolysis.
- Addition of detergents in general lead to decreased G250 detection levels in ELISA
- Truncation of the cDNA encoding for G250 antigen, followed by transient transfection in G250-negative cells, followed by immunohistochemical analysis revealed:

| cDNA construct | G250-reactivity |
|---|---|
| nt 1-1500 (aa 1-459), complete protein | + |
| nt 1-1218 (aa 1-406), G250 without transmembrane region | +/- |
| nt 1-1074 (aa 1-358) | - |
| nt 1-843 (aa 1-281) | - |
| nt 1-672 (aa 1-224) | - |
| nt 1-450 (aa 1-150) | - |

It was found that G250-reactivity was lost, even when large cDNA constructs were used.

To further delineate the G250 epitope, the NovaTope system (Novagen Inc.) was used, a system which ensures expression of all constructs ligated into this vector, i.e., all possible fragments are expressed, irrespective of reading frame. This allows identification of epitopes present in the middle part of a protein.

The cDNA encoding for the MN (G250) antigen was digested with DNAse I for 2 hours, resulting in fragments of approximately 50-200 nucleotides encoding polypeptides having a length of about 15-70 amino acids. After dA tailing, the fragments were inserted into the pScreen T vector, and the ligation mixture was added to competent E.coli DE3 bacteria for transformation. Bacteria were plated on selection plates. Bacterial clones were blotted onto nitrocellulose filters and screened with G250 antibody. In total 7 colonies were identified with MAbG250. G250 reactivity was weak. After further re-screening, only one bacterial clone appeared to react with MAbG250, albeit extraordinary very weak, and most likely due to background staining. Sequence analysis of the clone did not reveal any G250-sequences, but showed the presence of a truncated vector. Thus, the staining of the nitrocellulose blot was most likely the result of nonspecific background.

In a further experiment, cDNA encoding the MN (G250) antigen was digested with DNAse I, but now fragments of 200 - 600 nucleotides, encoding polypeptides of approximately 65 - 200 amino acids were isolated and cloned into the expression vector and expressed in bacteria. Separate bacterial colonies were screened for G250 reactivity.

Reactivity with G250 was not apparent. Blots were stained for extended times, after which a faint staining was observed with 6 bacterial isolates. The bacterial isolates only contained a truncated vector and an unrelated sequence.

Thus, no partial sequence of the MN antigen having strong reactivity with MAbG250 could be identified.

These results strongly suggest that the G250 epitope is a conformational epitope. Further, the G250 epitope on the MN antigen cannot be characterized by conventional methods even with considerable efforts.

### Example 3

### Development of a chemeric G250 IgG production cell line

### General strategy

In order to construct a mouse/human chimeric version of the murine G250 antibody, the variable region genes for the heavy and light chains, which determine the binding specificity of the antibody, were cloned from the G250 murine hybridoma and assembled in vitro with human constant region genes to generate mouse/human chimeric antibody genes. Expression of these chimeric genes in the appropriate cell line resulted in production of a chimeric antibody with the same binding characteristics as the original murine antibody, but with approximately 75% of the molecule comprising human sequences.

The strategy for cloning the variable regions for the heavy and light chain genes from the G250 hybridoma was based upon the linkage in the genome between the variable region and the corresponding J (joining) region for functionally rearranged (and expressed) immunoglobulin genes. J region DNA probes can be used to screen genomic libraries to isolate DNA linked to the J regions; DNA in the germline configuration (unrearranged) would also hybridize to J probes, but is not linked to a variable region sequence and can be identified by restriction enzyme analysis enzyme analysis of the isolated clones.

The cloning strategy, therefore, was to isolate variable regions from rearranged heavy and light chain genes using JH and JK probes. In addition, to assist in identifying and characterizing the correct genomic clones, cDNA clones corresponding to the heavy and light chain variable regions were also obtained from the mRNA produced in the G250 hybridoma. Those genomic clones that matched the G250 cDNA sequences were cloned into expression vectors containing human constant regions and transfected into mouse myeloma cells to determine if an antibody was produced. The antibody from producing cells was then tested for binding specificity compared to the murine G250 antibody.

### Cloning of G250 VH and VL cDNA

Total RNA was isolated from G250 hybridoma cell and used to generate specific VH and VL cDNA clones. First strand cDNA synthesis was performed using oligonucleotide primers specific for the murine kappa and IgG constant regions. After dG tailing, second strand synthesis was performed using C-tailed oligonucleotide primers. The heavy and light chain variable regions were amplified via polymerase chain reaction using specific 5' and 3' primers, and the amplification products were cloned into the plasmid vector pUC19. VH and VL clones were subjected to DNA sequence analysis to establish the base sequences of the putative G250 heavy and light chain variable regions. The sequencing results are shown in Fig. 1.

### Cloning of the G250 Variable Region Genes:

To clone the variable region genes, high molecular weight genomic DNA was isolated from G250 hybridoma cells by treatment of isolated nuclei with SDS and proteinase K followed by two phenol extractions and an ethanol precipitation.

Southern blot analysis using a J region probe for the heavy chain locus suggested that the G250 heavy chain was contained on a 2.3 kilobase (Kb) Eco RI DNA fragment. Accordingly, the Eco RI fragments from hybridoma DNA were fractionated on a 0.8% agarose gel and the size range of approximately 2-3 Kb fragments was eluted from the gel and ligated to the vector arms of the lambda bacteriophage vector Zap II (Stratagene, La Jolla California, USA). The ligation was packaged into phage particles in vitro using Gigapack Gold (Stratagene) and plated on E.coli LE392 cells at a density of approximately 20.000 plaques per 150 mm plate. The plaques were transferred to nitrocellulose filters and probed with a ³²P-labeled DNA probe (a 2.0 Kb Eco Ri-BamHi fragment containing the murine J3 and J4 exons).

For the light chain G250 variable region gene, a Southern blot analysis using a murine J region probe from the kappa locus indicated that the correct gene was located on a 5.5 Kb Hindlll fragment. Accordingly, G250 hybridoma DNA was digested with Hindlll and DNA fragments of 5-6 Kb were isolated from a 0.8% agarose gel and ligated to the arms of the bacteriophage lambda vector Charon 27. The ligated DNA was packaged into phage particles in vitro using Gigapack Gold (Stratagene), and plated on E.coli LE392 cells at a density of approximately 20.000 plaques per 150 mm plate. The plaques were transferred to nitrocellulose filters and probed with a ³²P-labeled DNA probe (a 2.7 Kb Hindlll fragment containing all five J kappa exons).

Positive clones for the heavy and light chain genes were isolated following at least 3 rounds of plaque purification using the J region probes to verify hybridization to the phage DNA at each stage of purification. Purified DNA from the isolated phage clones for the heavy and light genes was digested with EcoRI (heavy chain) or Hindlll (light chain) and fractionated on agarose gels. The appropriate size fragments (2.3 Kb for the heavy chain and 5.5 Kb for the light chain) were isolated from the gels, and subcloned into the plasmid vector pUC19. These subcloned DNA fragments were shown by restriction endonuclease mapping and DNA sequence analysis to contain variable region antibody genes that matched the structures of the cDNA clones originally obtained from the G250 hybridoma.

### Expression Plasmids

The 2.3 Kb Eco RI heavy chain variable region fragment was cloned into a suitable expression vector which contains the human G1 constant region and a gene which confers resistance to a selectable marker in mammalian cells. The 5.5. Kb Hindlll light chain fragment was cloned into a suitable expression vector which contains the human kappa constant region and a gene conferring resistance to a selectable marker in mammalian cells. The presence of the cloned fragments and their orientation in the expression vectors was determined by restriction enzyme mapping. The basic vectors have been used previously for the construction of chimeric antibodies (Sun, L. et al., (1987) PNAS 84, p. 214; Knight et al., (1993), Molecular Immunology 30, p. 1443-1453). Expression of the chimeric antibody genes is achieved using the natural immunoglobulin promoters present upstream of the cloned variable regions; downstream regulatory signals are provided by the elements associated with the constant region genes.

### Expression of the G250 Chimeric Antibody

The heavy and light chain expression vectors were used to co-transfect a non-producing mouse myeloma cell line using the technique of electroporation. An ELISA assay was used to screen clones for secreted antibody containing a human Fc region, and the highest producer (cG250) was chosen for further characterization.

The chimeric antibody was purified by chromatography on protein A-sepharose, and analyzed in a competition assay to verify that the antibody has the correct specificity. The cG250 antibody was found to be equally effective in competing with ¹²⁵I-labelled murine G250 for binding to A704 renal cell carcinoma cells (which express the G250 antigen) as the unlabeled murine G250 antibody. The chimeric G250 antibody, therefore, is similar in its binding characteristics to the original murine G250 antibody.

### Example 4

### Use of chimeric G250 antibody in a clinical study

### Administration protocol

Renal cell carcinoma patients, having metastases after surgery, were treated by administering 50 mg chimeric G250 antibody via 30 min. infusion once per week for 12 weeks.

### Efficacy conclusions:

In the present study durable stable disease for over six months was achieved in eight out of 32 patients (25%) of the patients. The median time to progression for all patients was 16 weeks (mean 26,67 weeks) with a range from 4 to 70 weeks. As of December 2001, the date of cut off, five patients are still free of progression (51 +, 56+, 60+, 64+, and 70 + weeks).

The importance of these findings is supported by the fact that most of these patients had documented progressive disease, for another two patients it is assumed that they were progressive at study entry. In addition, three of these patients were refractory to prior cytokine- and chemotherapy or had relapsed several years after cytokine treatment. Four patients had no prior therapy and were treated with the study drug immediately after diagnosis of metastases. At study entry three of these patients had a Karnofsky performance status (KPS) of 80%, two a KPS of 90%. Hemoglobin (Hgb) was below 10 g/dL in 5 patients. The median performance status of 90% (mean 91%) is identical to the group of patients who have not responded. The median Hgb of 9.8 g/dL (mean 10.97 g/dL) is also relatively low.

One patient achieved a complete objective remission. The objective response occurred late, more than six months after start of study therapy. Another patient experienced a relevant reduction of 59% in size of his target lesions. The response of both patients is ongoing at week 64 and 70, respectively, as evaluated in December 2001. The tumor regression occurred late, more than six months after start of study therapy. An explanation for the late response may be that the treatment duration with iv injections of the chimeric antibody was relatively short and a delayed immune response unrelated to ADCC developed.

The response is durable at more than one year and is still ongoing December 2001. The overall rate of clinical benefit (at least more than six months or objective response) is 25%. In addition it should be noted that at six months after start of study treatment, 87.5% (28/32) of the patients were still alive.

At the end of November 2001, an estimation of median survival was calculated to be at least 13.5 months.

At the end of January 2002, an estimation of median survival was calculated to be at least 15 months. This is significantly higher than the median survival of untreated patients (three months) and the median survival of patients treated with an FDA approved standard therapy, i.e. administration of high dose interleukin 2 (10-12 months).

Thus, it becomes evident that chimeric antibody G250 is a suitable therapeutic agent for the treatment of renal cell carcinoma. In a preferred embodiment the treatment comprises a surgical removal of the main tumor and a subsequent administration of G250, in order to eliminate metastases distributed throughout the body.

### SEQUENCE LISTING

<110> Wilex AG
<120> Hybridoma Cell Line
<130> 26037P WO_CG
<140> PCT/EP02/01282
   <141> 2002-02-07
<160> 2
<170> PatentIn Ver. 2.1
<210> 1
   <211> 357
   <212> DNA
   <213> Artificial Sequence
<220>
   <223> Description of Artificial Sequence:mouse/human chimeric antibody gene
<400> 1
   gacgtgaagc tcgtggagtc tgggggaggc ttagtgaagc ttggagggtc cctgaaactc 60
   tcctgtgcag cctctggatt cactttcagt aactattaca tgtcttgggt tcgccagact 120
   ccagagaaga ggctggagtt ggtcgcagcc attaatagtg atggtggtat cacctactat 180
   ctagacactg tgaagggccg attcaccatt tcaagagaca atgccaagaa caccctgtac 240
   ctgcaaatga gcagtctgaa gtctgaggac acagccttgt tttactgtgc aagacaccgc 300
   tcgggctact tttctatgga ctactggggt caaggaacct cagtcaccgt ctcctca    357
<210> 2
   <211> 321
   <212> DNA
   <213> Artificial Sequence
<220>
   <223> Description of Artificial Sequence:mouse/human chimeric antibody gene
<900> 2

## Claims

1. Use of a G250 antibody for the manufacture of a pharmaceutical formulation for the treatment of renal cell carcinoma patients after surgery, wherein the G250 antibody is produced by hybridoma cell DSM ACC 2526 or a progeny cell thereof, wherein the progeny cell has been obtained by transfer of genetic material encoding antibody G250 or at least the antigen-binding site thereof into a receptor cell.

2. The use of claim 1 for the treatment of metastases after tumor surgery.

3. Use of a G250 antibody for the manufacture of an agent for eliciting a delayed immune response in cancer patients more than six months after start of therapy in the treatment of renal cell carcinoma, wherein the G250 antibody is produced by hybridoma cell DSM ACC 2526 or a progeny cell thereof, wherein the progeny cell has been obtained by transfer of genetic material encoding antibody G250 or at least the antigen-binding site thereof into a receptor cell.

4. The use of any one of claims 1-3, wherein the antibody is a chimeric antibody, a humanized antibody, a fully human antibody, a bispecific antibody, a single chain antibody or a F(ab')₂, a Fab', or a Fab antibody fragment.

5. The use of claim 4, wherein the antibody is a chimeric G250 antibody as such.

6. The use of claim 5, wherein the pharmaceutical formulation comprises a chimeric G250 antibody coupled to a cytokine such as IL-2, TNF and/or GM-CSF.

7. The use of any one of claims 1-6, wherein the nucleic acid encoding the G250 antibody comprises a nucleic acid sequence as shown in SEQ ID NO: 1 and/or SEQ ID NO: 2.

8. The use of any one of claims 1-7, wherein the nucleic acid encoding the G250 antibody comprises
a) a nucleotide sequence encoding the complementarity determining regions CDR1, CDR2, and/or CDR3 of the heavy chain antigen-binding site as shown in Figure 1, designated H1-H3, and in SEQ ID NO: 1, and/or
b) a nucleotide sequence encoding the complementarity determining regions CDR1, CDR2, and/or CDR3 of the light chain antigen-binding site as shown in Figure 1, designated L1-L3, and in SEQ ID NO: 2.

9. The use of any one of claims 1-8, wherein the G250 antibody comprises an amino acid sequence encoded by a nucleotide sequence as shown in SEQ ID NO: 1 and/or SEQ ID NO: 2, and wherein the antibody selectively binds to the G250 antigen.

10. The use of any one of claims 1-9, wherein the G250 antibody comprises:
(a) a heavy chain variable region comprising complementarity determining regions CDR1, CDR2, and CDR3 as shown in Fig. 1 or at least the CDR3 region of the G250 heavy chain or a variant G250 CDR3 region having a sequence identity of at least 80%, preferably 90% to the original G250 heavy chain CDR3 region as shown in Fig. 1 on the amino acid level and/or
(b) a light chain variable region comprising complementarity determining regions CDR1, CDR2, and CDR3 as shown in Fig. 1 or at least the CDR3 region of the G250 light chain or a variant G250 CDR3 region having a sequence identity of at least 80%, preferably 90% to the original G250 light chain CDR3 region as shown in Fig. 1 on the amino acid level,
wherein said variant CDR3 region of the heavy chain and/or of the light chain has equivalent antigen-binding characteristics compared to the the original CDR3 region.

11. The use of any one of claims 1-10, wherein the patients have progressive disease.

12. The use of any one of claims 1-11, wherein the patients were refractory to prior cytokine- or chemotherapy, or had relapsed after cytokine treatment.

## Patentansprüche

1. Verwendung eines G250 Antikörpers zur Herstellung einer pharmazeutischen Formulierung zur Behandlung von Patienten mit Nierenzellkarzinom nach chirurgischer Entfernung, wobei der G250 Antikörper von der Hybridomzelle DSM ACC 2526 oder einer Nachkommenzelle davon produziert wird, wobei die Nachkommenzelle durch Transfer von genetischem Material, welches für den Antikörper G250 oder zumindest einer Antigen-bindenden Stelle davon kodiert, in eine Rezeptorzelle erhalten worden ist.

2. Verwendung gemäß Anspruch 1 zur Behandlung von Metastasen nach chirurgischer Tumorentfernung.

3. Verwendung eines G250 Antikörpers zur Herstellung eines Wirkstoffs, um eine verzögerte Immunreaktion in Krebspatienten mehr als sechs Monate nach Beginn der Therapie in der Behandlung von Nierenzellkarzinom auszulösen, wobei der G250 Antikörper von der Hybridomzelle DSM ACC 2526 oder einer Nachkommenzelle davon produziert wird, wobei die Nachkommenzelle durch Transfer von genetischem Material, welches für den Antikörper G250 oder zumindest einer Antigen-bindenden Stelle davon kodiert, in eine Rezeptorzelle erhalten worden ist.

4. Verwendung gemäß einem der Ansprüche 1-3, wobei der Antikörper ein chimärer Antikörper, ein humanisierter Antikörper, ein vollhumanisierter Antikörper, ein bispezifischer Antikörper, ein Einzelketten-Antikörper oder ein F(ab')₂-, ein Fab'- oder ein Fab-Antikörperfragment ist.

5. Verwendung gemäß Anspruch 4, wobei der Antikörper ein chimärer Antikörper als solcher ist.

6. Verwendung gemäß Anspruch 5, wobei die pharmazeutische Formulierung einen chimären G250 Antikörper, der an ein Cytokin, wie zum Beispiel IL-2, TNF und/oder GM-CSF, gebunden ist, umfasst.

7. Verwendung gemäß einem der Ansprüche 1-6, wobei die Nukleinsäure, die für den G250 Antikörper kodiert, eine Nukleinsäuresequenz wie in SEQ ID NO: 1 und/oder SEQ ID NO: 2 gezeigt, umfasst.

8. Verwendung gemäß einem der Ansprüche 1-7, wobei die Nukleinsäure, die für den G250 Antikörper kodiert, umfasst
a) eine Nukleotidsequenz, die für die Komplementaritätsdeterminierenden Regionen CDR1, CDR2 und/oder CDR3 der Antigenbindungsstelle der schweren Kette kodiert, wie in Figur 1, bezeichnet als H1-H3, und in SEQ ID NO: 1 gezeigt und/oder
b) eine Nukleotidsequenz, die für die Komplementaritätsdeterminierenden Regionen CDR1, CDR2 und/oder CDR3 der Antigenbindungsstelle der leichten Kette kodiert, wie in Figur 1, bezeichnet als L1-L3, und in SEQ ID NO: 2 gezeigt.

9. Verwendung gemäß einem der Ansprüche 1-8, wobei der G250 Antikörper eine Aminosäuresequenz umfasst, die durch eine Nukleotidsequenz, wie in SEQ ID NO: 1 und/oder SEQ ID NO: 2 gezeigt, kodiert wird, und wobei der Antikörper selektiv an das G250 Antigen bindet.

10. Verwendung gemäß einem der Ansprüche 1-9, wobei der G250 Antikörper umfasst
(a) eine variable Region der schweren Kette, umfassend Komplementaritäts-determinierende Regionen CDR1, CDR2 und CDR3, wie in Figur 1 gezeigt, oder zumindest die CDR3-Region der G250 schweren Kette oder eine abweichende G250 CDR3-Region, die eine Sequenzidentität von zumindest 80 %, bevorzugt 90 % zur ursprünglichen G250 CDR3-Region der schweren Kette, wie in Figur 1 gezeigt, auf der Aminosäureebene aufweist und/oder
(b) eine variable Region der leichten Kette, umfassend Komplementaritäts-determinierende Regionen CDR1, CDR2 und CDR3, wie in Figur 1 gezeigt, oder zumindest die CDR3-Region der G250 leichten Kette oder eine abweichende G250 CDR3-Region, die eine Sequenzidentität von zumindest 80 %, bevorzugt 90 % zur ursprünglichen G250 CDR3-Region der leichten Kette, wie in Figur 1 gezeigt, auf der Aminosäureebene aufweist,
wobei die abweichende CDR3-Region der schweren Kette und/oder der leichten Kette die äquivalenten Antigenbindungseigenschaften im Vergleich zur ursprünglichen CDR3-Region aufweist.

11. Verwendung gemäß einem der Ansprüche 1-10, wobei die Patienten eine fortschreitende Krankheit aufweisen.

12. Verwendung gemäß einem der Ansprüche 1-11, wobei die Patienten Renitenz gegenüber einer früheren Cytokin- oder Chemotherapie aufwiesen oder im Anschluss an einer Cytokinbehandlung einen Rückfall hatten.

## Revendications

1. Utilisation d'un anticorps G250 pour la fabrication d'une formulation pharmaceutique destinée au traitement de patients présentant un carcinome des cellules rénales après une chirurgie, où l'anticorps G250 est produit par la cellule d'hybridome DSM ACC 2526 ou une cellule fille de celle-ci, où la cellule fille a été obtenue par un transfert du matériel génétique codant pour l'anticorps G250 ou au moins du site de liaison à l'antigène de celui-ci dans une cellule réceptrice.

2. Utilisation selon la revendication 1 destinée au traitement des métastases après la chirurgie d'une tumeur.

3. Utilisation d'un anticorps G250 pour la fabrication d'un agent destiné à provoquer une réponse immunitaire retardée chez des patients cancéreux plus de six mois après le début d'une thérapie dans le traitement du carcinome des cellules rénales, où l'anticorps G250 est produit par la cellule d'hybridome DSM ACC 2526 ou une cellule fille de celle-ci, où la cellule fille a été obtenue par un transfert du matériel génétique codant pour l'anticorps G250 ou au moins du site de liaison à l'antigène de celui-ci dans une cellule réceptrice.

4. Utilisation selon l'une quelconque des revendications 1 à 3, dans laquelle l'anticorps est un anticorps chimérique, un anticorps humanisé, un anticorps totalement humain, un anticorps bispécifique, un anticorps simple chaîne ou un fragment d'anticorps F(ab')₂, Fab' ou Fab.

5. Utilisation selon la revendication 4, dans laquelle l'anticorps est un anticorps G250 chimérique en tant que tel.

6. Utilisation selon la revendication 5, dans laquelle la formulation pharmaceutique comprend un anticorps G250 chimérique couplé à une cytokine telle que l'IL-2, le TNF et/ou le GM-CSF.

7. Utilisation selon l'une quelconque des revendications 1 à 6, dans laquelle l'acide nucléique codant pour l'anticorps G250 comprend une séquence d'acide nucléique telle que montrée dans SEQ ID NO: 1 et/ou SEQ ID NO: 2.

8. Utilisation selon l'une quelconque des revendications 1 à 7, dans laquelle l'acide nucléique codant pour l'anticorps G250 comprend
a) une séquence de nucléotides codant pour les régions déterminant la complémentarité CDR1, CDR2 et/ou CDR3 du site de liaison à l'antigène de chaîne lourde telles que montrées sur la Figure 1, désignées H1-H3, et en SEQ ID NO: 1, et/ou
b) une séquence de nucléotides pour les régions déterminant la complémentarité CDR1, CDR2 et/ou CDR3 du site de liaison à l'antigène de chaîne légère telles que montrées sur la Figure 1, désignées L1-L3, et en SEQ ID NO: 2.

9. Utilisation selon l'une quelconque des revendications 1 à 8, dans laquelle l'anticorps G250 comprend une séquence d'acides aminés codée par une séquence de nucléotides telle que montrée dans SEQ ID NO: 1 et/ou SEQ ID NO: 2, et où l'anticorps se lie sélectivement à l'antigène G250.

10. Utilisation selon l'une quelconque des revendications 1 à 9, dans laquelle l'anticorps G250 comprend :
(a) une région variable de chaîne lourde comprenant les régions déterminant la complémentarité CDR1, CDR2 et/ou CDR3 telles que montrées sur la Figure 1 ou au moins la région CDR3 de la chaîne lourde de G250 ou une région CDR3 de G250 variante présentant une identité de séquence d'au moins 80 %, de préférence 90 %, avec la région CDR3 de la chaîne lourde de G250 d'origine telle que montrée sur la Figure 1 au niveau des acides aminés et/ou
(b) une région variable de chaîne légère comprenant les régions déterminant la complémentarité CDR1, CDR2 et/ou CDR3 telles que montrées sur la Figure 1 ou au moins la région CDR3 de la chaîne légère de G250 ou une région CDR3 de G250 variante présentant une identité de séquence d'au moins 80 %, de préférence 90 %, avec la région CDR3 de la chaîne légère de G250 d'origine telle que montrée sur la Figure 1 au niveau des acides aminés,
où ladite région CDR3 variante de la chaîne lourde et/ou de la chaîne légère possède des caractéristiques de liaison à l'antigène équivalentes par comparaison à la région CDR3 d'origine.

11. Utilisation selon l'une quelconque des revendications 1 à 10, dans laquelle les patients présentent une maladie évolutive.

12. Utilisation selon l'une quelconque des revendications 1 à 11, dans laquelle les patients étaient réfractaires à un traitement antérieur par une cytokine ou une chimiothérapie, ou ont présenté une rechute suite à un traitement par une cytokine.
